Europäisches Patentamt

European Patent Office

Office européen des brevets

(11) Veröffentlichungsnummer: **0 433 717 A1**

# EUROPÄISCHE PATENTANMELDUNG

(21) Anmeldenummer: 90122692.8

(22) Anmeldetag: 28.11.90

(51) Int. Cl.5: **A61B 17/34, A61M 25/06**

(30) Priorität: 19.12.89 DE 8914941 U

(43) Veröffentlichungstag der Anmeldung:
26.06.91 Patentblatt 91/26

(84) Benannte Vertragsstaaten:
AT BE CH DE DK ES FR GB GR IT LI NL SE

(71) Anmelder: **B. Braun Melsungen AG**
**Carl-Braun Strasse**
**W-3508 Melsungen(DE)**

(72) Erfinder: **Haindl, Hans-Günter, Dr. Dipl.-Ing.**
**Schöne Aussicht 4**
**W-3508 Melsungen(DE)**
Erfinder: **Schmidt, Klaus Joachim**
**Brandenburger Strasse 16**
**W-3501 Ahnatal(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) Punktionsbesteck.

(57) Das erfindungsgemäße Punktionsbesteck mit einem großlumigen Kunststoffkapillar erlaubt eine schonende Punktion mit geringem Initialpunktionstrauma. Dies wird dadurch erreicht, daß die Ansätze (13,16,21) der Stahlkanüle (11), der Dilatatorhülse (15) und des Kunststoffkapillars (20) koaxial lösbar zusammengesteckt und die Dilatatorhülse (15) auf der Stahlkanüle (11) und das Kunststoffkapillar (20) auf der Dilatatorhülse (15) in Richtung der Spitze des jeweiligen Teiles vorschiebbar sind.

FIG.1

## PUNKTIONSBESTECK

Die Erfindung bezieht sich auf ein Punktionsbesteck, nach dem Oberbegriff des Patentanspruches 1.

Es ist ein Punktionsbesteck bekannt (DE-U-7 129 912), bei dem das Kunststoffkapillar die in diesem steckende Stahlkanüle im wesentlichen spaltfrei umschließt und nach der Punktion eines Blutgefäßes mit Hilfe der Spitze der Stahlkanüle und Vorschieben des Kunststoffkapillars in das Blutgefäß die Stahlkanüle herausgezogen wird. Das Kunststoffkapillar wird an dem Patienten festgelegt und es wird an den Kapillaransatz eine Infusionsleitung oder dergleichen angekuppelt. Ferner ist es üblich, das Kunststoffkapillar eines solchen Punktionsbesteckes zum Einbringen von Gefäßkathetern zu benutzen. Zu diesem Zweck muß allerdings ein verhältnismäßig großlumiges Kunststoffkapillar benutzt werden und entsprechend großen Durchmesser hat dabei die Stahlkanüle. Es hat sich gezeigt, daß die Anwender sich scheuen, mit einer großlumigen Stahlkanüle in zentrale Blutgefäße zu stechen, da zu befürchten ist, daß bei einer Fehlpunktion, z.B. in eine Arterie oder in den Pleuraspalt, von dem großen Kaliber der Stahlkanüle erhöhte Gefahren ausgehen. Aus diesem Grunde wird häufiger das Einführen eines Katheters über einen Führungsdraht praktiziert, was jedoch umständlicher ist als das "Einschleusen" des Katheters durch ein Kunststoffkapillar.

Mit einem Führungsdraht arbeitet auch das nicht der Punktion dienende medizinische Einführungsbesteck nach DE 38 14 618 C1, das aus einer Innenhülse mit konisch verjüngtem Endabschnitt, einer diese umgebenden Zwischenhülse und einer auf der Zwischenhülse steckenden Außenhülse besteht, die alle relativ zueinander axial verschiebbar sind. Die Innenhülse weist hinter dem konischen Endabschnitt eine Außenschulter auf, gegen die der proximale Rand der Außenhülse anstößt. Durch Vorschieben der Zwischenhülse in Richtung der Außenschulter wird die Außenhülse elastisch aufgeweitet und von der Außenschulter der Innenhülse abgehoben, so daß diese gemeinsam mit der Zwischenhülse durch die Außenhülse zurückgezogen werden kann und die größer dimensionierte Außenhülse in dem Punktionsloch verbleibt. Mit diesem Einführungsbesteck wird das in einem gesonderten Schritt mit einer Stahlkanüle ausgebildete Punktionsloch, aus dem ein Führungsdraht nach außen ragt, auf dem das Einführungsbesteck entlanggleitet, stufenlos aufgeweitet. Die aufeinanderfolgenden Schritte der separaten Punktion, der Verlegung eines Führungsdrahtes und der Einbringung des Einführungsbesteckes bedeuten aber erhöhte Belastung und Sicherheitsrisiken für den Patienten.

Auch wird der Anwender durch die Schrittfolge unerwünscht beansprucht.

Das Punktionsbesteck nach dem Oberbegriff des Patentanspruches 1 ist in US-A-4 099 528 beschrieben. Dieses bekannte Punktionsbesteck ist Teil einer zweilumigen Kathetervorrichtung, bei der der Kapillaransatz des Kunststoffkapillars als Y-Stück gestaltet ist. Die Dilatatorhülse befindet sich in einem ringförmigen Spaltraum zwischen einem äußeren und einem inneren Kunststoffkapillar, die zueinander unverschieblich an dem Y-Stück befestigt sind. In dem inneren Kunststoffkapillar steckt die Stahlkanüle. Die Dilatatorhülse ist geknickt und ragt aus dem abgewinkelten Schenkel des Y-Stükkes heraus. Sie ist axial breit geschlitzt, damit sie an dem inneren Kunststoffkapillar vorbei bewegbar ist, wenn sie in der Vorrichtung verschoben wird. Die Spitzen der ineinandersteckenden rohrförmigen Teile bilden eine bei der Punktion unveränderliche Einheit mit dicker koni scher Spitze, deren Durchmesser sich von dem Außendurchmesser der Stahlkanüle zum Außendurchmesser des äußeren Kunststoffkapillars gleichmäßig vergrößert. Beim Vorschub der konischen Spitze der Einheit wird das Punktionsloch stark aufgeweitet und bis zum Eintritt des äußeren Kunststoffkapillars in das Punktionsloch ist eine große Eindringlänge der Spitze notwendig, wodurch sich die Gefahr einer Läsion der der Einstichstelle gegenüberliegenden Wand eines Körperhohlraumes ergibt. Im übrigen ist das gesamte Punktionsbesteck unhandlich und es ist zu befürchten, daß beim Herausziehen der Dilatatorhülse beide Kunststoffkapillare sich unkontrolliert bewegen und Gewebeschäden hervorrufen. Die eingangs erwähnten Risiken bei einer Fehlpunktion mit großlumiger Kanüle sind bei diesem Punktionsbesteck entsprechend vorhanden.

Der Erfindung liegt die Aufgabe zugrunde, ein Punktionsbesteck zu schaffen, das bei Ausrüstung mit einem großlumigen Kunststoffkapillar eine schonende Punktion mit geringem Initialpunktionstrauma erlaubt.

Diese Aufgabe wird erfindungsgemäß durch die Merkmale des kennzeichnenden Teils des Patentanspruches 1 gelöst.

Ein solches Punktionsbesteck ermöglicht das Punktieren zentraler Blutgefäße mit einer verhältnismäßig dünnen Stahlkanüle, so daß das Punktionstrauma primär klein ist. Erst wenn mit dieser dünnen Stahlkanüle das Blutgefäß punktiert ist und der Punktionserfolg durch Aspiration bestätigt worden ist, wird die von dem Kunststoffkapillar umgebene dicke Dilatatorhülse auf der Stahlkanüle vorgeschoben, wobei das Punktionsloch auf das Außenmaß der Dilatatorhülse und anschließend des Kunststoffkapillars erweitert wird. Wenn das Kunststoffkapillar weit genug in das Blutgefäß vorgeschoben worden ist, werden die Stahlkanüle und die Dilatatorhülse aus dem Kunststoffkapillar herausgezogen, so daß nur noch das großlumige Kunststoffkapillar in dem Blutgefäß verbleibt. Nunmehr kann durch das Kunststoffkapillar ein Katheter in das Blutgefäß eingeschoben werden. Alternativ ist es möglich, an den Kapillaransatz eine Infusionsleitung anzuschließen und das große Lumen des Kunststoffkapillars zur Schnellinfusion großer Flüssigkeitsmengen einzusetzen.

Vorteilhafterweise fällt die Spitze der Dilatatorhülse außen konisch gegen den Außenumfang der Stahlkanüle ab und liegt innen dicht an der Stahlkanüle an. Auf diese Weise wird das Einschieben der Spitze der Dilatatorhülse in das kleine Punktionsloch erleichtert und es wird vermieden, daß Blut zwischen Stahlkanüle und Dilatatorhülse nach außen gelangt. Diese Abdichtung an der Spitze der Dilatatorhülse ist um so wichtiger, als vorzugsweise die Innenfläche der Dilatatorhülse in ihrem Verlauf von der Spitze zum Hülsenansatz von dem Außenumfang der Stahlkanüle abgehoben ist. Zweck dieser die Wandstärke verringernden Maßnahme ist es, die Flexibilität der Dilatatorhülse zu erhöhen, die aus Material besteht, das hart genug sein muß, um ein schiebendes Dilatieren des Stichkanals zu ermöglichen. Eine gewisse Flexibilität der Dilatatorhülse ist dagegen wichtig, damit sie sich nach dem Vorschieben über die Spitze der Stahlkanüle der Krümmung des Blutgefäßes anpassen kann, in das das Kunststoffkapillar eingebracht werden soll.

Die Wandstärke des Kunststoffkapillars ist in Bezug auf diejenige der Dilatatorhülse sehr gering. Da das Kunststoffkapillar in dem Einstichloch keinerlei Erweiterungsfunktion hat, kann eine für eine langzeitige Verlegung in dem Blutgefäß günstige Weichheit beliebig vorgesehen sein.

Der Kapillaransatz, der Kanülenansatz und der Hülsenansatz sind jeweils mit einer quergerichteten Griffplatte ausgestattet, die das Zusammenstecken der Ansätze zur gemeinsamen Handhabung der drei Teile des Punktionsbesteckes erleichtern bzw. ein einfaches Trennen der Ansätze zur separaten Verschiebung der rohrförmigen Teile in Bezug aufeinander ermöglichen.

Die Dilatatorhülse mit dem Kunststoffkapillar ist um die Länge des Überstandes der Spitze der Stahlkanüle über die Dilatatorhülse in Richtung der Stahlkanülenspitze vorschiebbar und es ist das Kunststoffkapillar um die Länge des Überstandes der Spitze der Dilatatorhülse über das Kunststoffkapillar in Richtung der Dilatatorhülsenspitze vorschiebbar. Die jeweiligen Verschiebungen um die korrekten Streckenlängen werden vorzugsweise durch Markierungen an der Stahlkanüle und der Dilatatorhülse angezeigt, die nach der Verschiebung des jeweils darüber befindlichen Teiles in Erscheinung treten.

In der Zeichnung ist ein Ausführungsbeispiel der Erfindung schematisch dargestellt.

Es zeigen:

Fig. 1 einen Längsschnitt durch das Punktionsbesteck im Punktionszustand, und

Fig. 2 die Anordnung nach Fig. 1 bei der Einführung des Kunststoffkapillars in ein Blutgefäß.

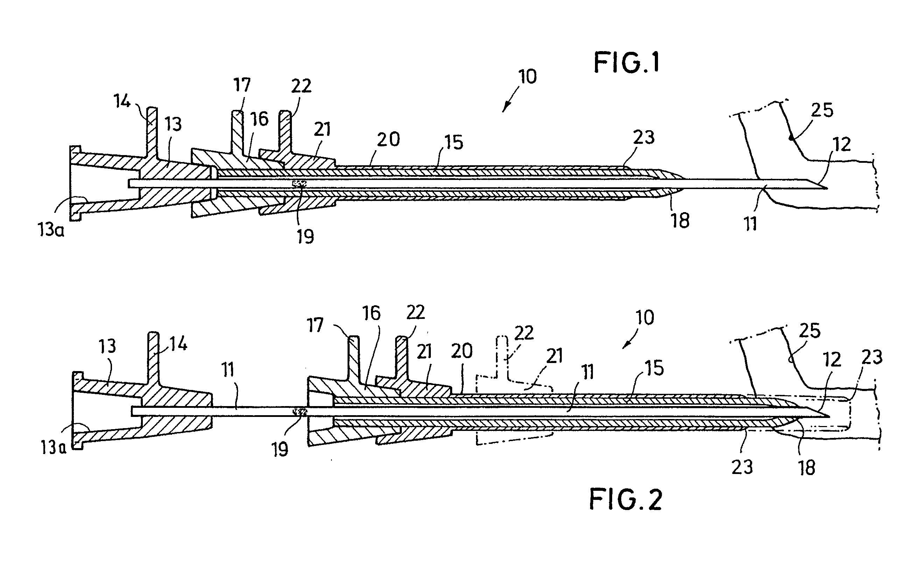

Ein Punktionsbesteck 10 besteht aus einer langen dünnen geraden Stahlkanüle 11 mit angeschliffener Spitze 12, einer die Stahlkanüle 11 umgebenden Dilatatorhülse 15 mit absatzloser Außenfläche und einem Kunststoffkapillar 20, das die Dilatatorhülse 15 umgibt. Die drei Teile 11,15 und 20 stecken axial verschiebbar konzentrisch ineinander und ihre anwendungsbereite gegenseitige Position (Fig. 1) wird durch das Zusammenstecken der Konen eines Kanülenansatzes 13 mit einem Hülsenansatz 16 und des Hülsenansatzes 16 mit einem Kapillaransatz 21 erreicht. In diesem Zustand wird das Punktionsbesteck steril geliefert. Die Ansätze 13,16,21 haben quergerichtete Griffplatten 14,17 und 22, die zueinander parallel und auf gleicher gerader Linie liegend ausgerichtet sind.

Das Kunststoffkapillar 20 ist dünnwandig und flexibel. Seine Öffnung 23 hat einen leicht eingezogenen Rand, der gegen den Außenumfang der Dilatatorhülse 15 abdichtend anliegt. Es hat großen Durchmesser und definiert mit dem Außenumfang der Stahlkanüle 11 einen kreiszylindrischen Spaltraum, in dem sich die Dilatatorhülse 15 befindet, deren Wandstärke beträchtlich größer als diejenige des Kunststoffkapillars 20 ist. Die Spitze 18 der Dilatatorhülse 15 steht über die Öffnung 23 des Kunststoffkapillars 20 ein Stück vor - ist jedoch zu der Spitze 12 der Stahlkanüle 11 zurückgesetzt. Die Spitze 18 der Dilatatorhülse 15 verjüngt sich gegen den Außenumfang der Stahlkanüle 11 konisch und der innere Rand ihrer Öffnung umschließt die Stahlkanüle 11. Die Stahlkanüle 11 und die Dilatatorhülse 15 sind einander so angeformt, daß nur ein minimaler Spalt zwischen der Dilatatorspitze 18 und dem Außenumfang der Stahlkanüle 11 besteht. Zur Erhöhung der Flexibilität der an sich steifen Dilatatorhülse 15 ist ihre Innenfläche in ihrem Verlauf von der Spitze 18 zum Hülsenansatz 16 von dem Außenumfang der Stahlkanüle 11 abgehoben, d.h. die Wandstärke der Dilatatorhülse 15 ist über den Hauptteil ihrer Länge geringer als die radiale Abmessung des zylindrischen Spaltraumes zwischen Stahlkanüle 11 und Kunststoffkapillar 20. Der Überstand der Stahlkanüle 11 über die Mündung der Spitze 18 der Dilatatorhülse 15 ist verhältnismäßig lang. Ein von dem Kanülenansatz 13 ausgehendes Stück von dem Stahlkanülen-Überstand entsprechender Länge ist auf der Stahlkanüle 11 bei 19 markiert.

Die Anwendung des Punktionsbesteckes zum Einführen des Kunststoffkapillars 20 entweder als Hilfe zum Einbringen eines Katheters in ein Blutgefäß oder zur direkten Verwendung als großlumige Schnellinfusionskanüle wird nachfolgend erläutert.

Die Punktion eines Blutgefäßes 25 erfolgt mit dem kompletten Punktionsbesteck 10, an das über einen Innenkonus 13a des Kanülenansatzes 13 eventuell eine Spritze angesetzt ist. Dabei erreichen die Dilatatorhülse 15 und das Kunststoffkapillar 20 nur den Subcutanbereich und lediglich die dünne Stahlkanüle 11 wird in den kritischen Bereich des Gefäßverlaufes vorgeschoben.

Sobald durch Rückfluß von Blut die korrekte Lage der Stahlkanüle 11 sichergestellt ist und durch den Druck des Blutes oder durch seine Farbe auch die venöse Lage sicher ist, wird die Stahlkanüle 11 mit Hilfe ihres Kanülenansatzes 13 an dem Patienten fixiert und es wird der Hülsenansatz 16 von dem Kanülenansatz 13 getrennt. Die Dilatatorhülse 15 mit dem auf dieser sitzenden Kunststoffkapillar 20 wird mit Hilfe der gemeinsam erfaßten Griffplatten 17,22 auf der Stahlkanüle 11 in Richtung ihrer Spitze 12 vorgeschoben. Das Erscheinen der Markierung 19 signalisiert dem Anwender, wenn die Spitze 18 der Dilatatorhülse 15 sich in dem Blutgefäß 25 befindet (Fig. 2).

Wenn dies der Fall ist, kann der Hülsenansatz 16 der Dilatatorhülse 15 an dem Patienten fixiert werden und es kann das Kunststoffkapillar 20 nach Trennung des Kapillaransatzes 21 von dem Hülsenansatz 16 über die Dilatatorhülse 15 in Richtung der Spitze 12 der Stahlkanüle 11 weiter in das Blutgefäß 25 vorgeschoben werden. Dies ist in Fig. 2 gestrichelt angedeutet. Auch hierbei kann die Vorschubstrecke des Kunststoffkapillars 20 sichtbar gemacht werden durch eine Markierung auf der Dilatatorhülse 15, die sichtbar wird, wenn sie von dem Kunststoffkapillar 20 freikommt.

Wenn das Kunststoffkapillar 20 weit genug in das Blutgefäß 25 eingeschoben worden ist, werden die Stahlkanüle 11 und die Dilatatorhülse 15 aus dem Kunststoffkapillar 20 axial herausgezogen. Durch das freie Lumen des Kunststoffkapillars 20 kann nun ein Katheter in das Blutgefäß 25 eingebracht werden oder es kann durch Anschluß einer Infusionsleitung an den Kapillaransatz 21 die Infusion großer Flüssigkeitsmengen in das Blutgefäß 25 erfolgen.

**Ansprüche**

1. Punktionsbesteck, bestehend aus einem Kunststoffkapillar (20) mit Kapillaransatz (21), einer Stahlkanüle (11) mit Kanülenansatz (13), deren Spitze (12) über das Kunststoffkapillar (20) vorsteht und einer in dem Kunststoffkapillar (20) axial verschiebbaren Dilatatorhülse (15), die einen Hülsenansatz (l6) trägt und deren Spitze (18) sich vor der Öffnung (23) des Kunststoffkapillars (20) und hinter der Spitze (12) der Stahlkanüle (11) befindet,

   **dadurch gekennzeichnet,**

   daß die Ansätze (13,16,21) der Stahlkanüle (11), der Dilatatorhülse (15) und des Kunststoffkapillars (20) koaxial lösbar zusammengesteckt sind und daß die Dilatatorhülse (15) auf der Stahlkanüle (11) und das Kunststoffkapillar (20) auf der Dilatatorhülse (15) in Richtung der Spitze des jeweiligen Teiles vorschiebbar sind.

2. Punktionsbesteck nach Anspruch 1, dadurch gekennzeichnet, daß die Spitze (18) der Dilatatorhülse (15) außen konisch gegen den Außenumfang der Stahlkanüle (11) abfällt und dicht an der Stahlkanüle (11) anliegt und daß die Innenfläche der Dilatatorhülse (15) in ihrem Verlauf von der Spitze (18) zum Hülsenansatz (16) von dem Außenumfang der Stahlkanüle (11) abgehoben ist.

3. Punktionsbesteck nach Anspruch 1 oder 2, dadurch gekennzeichnet, daß der Kapillaransatz (21), der Kanülenansatz (l3) und der Hülsenansatz (16) jeweils mit einer quergerichteten Griffplatte (22,14,17) ausgestattet sind.

4. Punktionsbesteck nach einem der Ansprüche 1-3, dadurch gekennzeichnet, daß die Dilatatorhülse (15) mit dem Kunststoffkapillar (20) um die Länge des Überstandes der Spitze der Stahlkanüle (11) über die Dilatatorhülse (15) in Richtung der Stahlkanülenspitze (12) vorschiebbar ist und daß das Kunststoffkapillar (20) um die Länge des Überstandes der Spitze der Dilatatorhülse (15) über das Kunststoffkapillar in Richtung der Dilatatorhülsenspitze (18) vorschiebbar ist.

5. Punktionsbesteck nach einem der Ansprüche 1-4, dadurch gekennzeichnet, daß der Außendurchmesser der Dilatatorhülse (15) beträchtlich größer als derjenige der Stahlkanüle (11) ist.

## FIG.1

## FIG.2

EP 0 433 717 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 182 666 (TECHNOLOGICAL SUPPLY INC.)<br>* Seite 2, Zeilen 9-15; Seite 3, Zelen 12-32; Seite 5, Zeilen 2-8; Figuren 1,8-10 *<br>— — — | 1-5 | A<br>61 B 17/34<br>A 61 M 25/06 |
| X | US-A-3 565 074 (FOTI)<br>* Zusammenfassung; Seite 2, Zeile 72 - Seite 3, Zeile 51; Anspruch 1; Figuren 1-7 *<br>— — — | 1,2,4,5 | |
| A | SOVIET INVENTIONS ILLUSTRATED, Woche 83-09, Sektion P31, Klasse P31, Zusammenfassung Nr. 83-D0219K/09, Nr. 83-039604, Derwent Publications Ltd, Londen, GB;<br>& SU-A-921 554 (TSELINOGRAD MED. INS.) 23-04-1982<br>— — — | 1 | |
| A | DE-U-8 622 507 (B. BRAUN MELSUNGEN AG)<br>* Seite 5, Zeile 26 - Seite 6, Zeile 2; Figur 1 *<br>— — — | 1 | |
| A | US-A-3 312 220 (EISENBERG)<br>* Spalte 2, Zeile 71 - Spalte 3, Zeile 24; Figuren 1-4,6 *<br>— — — — — | 3 | |

| | | | RECHERCHIERTE SACHGEBIETE (Int. Cl.5) |
|---|---|---|---|
| | | | A 61 B<br>A 61 M |

Der vorliegende Recherchenbericht wurde für alle Patentansprüche erstellt

| Recherchenort | Abschlußdatum der Recherche | Prüfer |
|---|---|---|
| Den Haag | 01 März 91 | ZEINSTRA H.S.J.H. |